# EUROPEAN PATENT APPLICATION

(11) **EP 4 606 783 A1**
(43) Date of publication of application: **27.08.2025**
(21) Application number: 23879978.7
(22) Date of filing: 10.08.2023
(51) Int. Cl.: C07C 51/487, C07C 59/01, C07C 59/08, C08J 11/24, C08J 11/16

(54) **METHOD FOR PREPARING HYDROXYALKANOIC ACID AND LACTIC ACID**

(30) Priority: 17.10.2022 KR 20220133485
(71) Applicant: LG Chem, Ltd., Yeongdeungpo-gu Seoul 07336 (KR)
(72) Inventor: KIM, Si Min, Daejeon 34122 (KR); AHN, Sangbum, Daejeon 34122 (KR); KANG, Donggyun, Daejeon 34122 (KR); JUNG, Woochul, Daejeon 34122 (KR)
(74) Representative: Goddar, Heinz J.
(86) International application number: PCT/KR2023/011848
(87) International publication number: WO 2024/085395

(57) **Abstract**

The present invention provides a manufacturing method of hydroxyalkanoic acid and lactic acid, the method comprising the steps of: hydrolyzing a hydroxyalkanoate-lactide copolymer in the presence of an alkali metal salt to produce a hydroxyalkanoic acid inorganic salt and a lactic acid inorganic salt, and purifying the hydroxyalkanoic acid inorganic salt and the lactic acid inorganic salt by electrodialysis to manufacture hydroxyalkanoic acid and lactic acid.

## Description

### FIELD OF THE INVENTION

### CROSS-REFERENCE TO RELATED APPLICATION(S)

This application claims the benefit of Korean Patent Application No. 10-2022-0133485 filed on October 17, 2022 in the Korean Intellectual Property Office, the disclosure of which is incorporated herein by reference in its entirety.

The present invention relates to a manufacturing method of hydroxyalkanoic acid and lactic acid by hydrolyzing a hydroxyalkanoate-lactide copolymer.

### BACKGROUND OF THE INVENTION

Plastics are inexpensive and durable materials, which can be used to produce a variety of products that find use in a wide range of applications. As a consequence, the manufacturing of plastics has increased dramatically over the last decades. Moreover, more than 50% of these plastics are used for single-use disposable applications, such as packaging, agricultural films, disposable consumer items or for short-lived products that are discarded within a year of manufacturing. Because of the durability of the polymers involved, substantial quantities of plastics are piling up in landfill sites and in natural habitats worldwide, generating increasing environmental problems. Even degradable and biodegradable plastics may persist for decades depending on local environmental factors, like levels of ultraviolet light exposure, temperature, presence of suitable microorganisms, etc.

In this regard, different solutions have been studied to reduce economic and environmental impacts correlated to the accumulation of plastic, from plastic degradation to plastic recycling.

As an example, polyethylene terephthalate(PET) is the most closed-loop recycled plastic (a system that processes and recycles waste generated from the manufacturing process). PET wastes (mainly bottles) are collected, sorted, pressed into bales, crushed, washed, chopped into flakes, melted and extruded in pellets and offered for sale. However, such plastic recycling processes are adapted to plastic items containing only PET, and thus need a prior extensive sorting.

Another potential process for recycling plastic consists of chemical recycling allowing recovering the chemical constituents of the polymer. The resulting monomers, after purification, may be used to re-produce plastic items. For example, as a plant-derived resin obtained from plants such as corn, polylactic acid has biodegradable properties and also has excellent tensile strength and elastic modulus, and is used for disposable or short-term products, and therefore, a chemical regeneration method for recycling it is needed.

### DETAILED DESCRIPTION OF THE INVENTION

### TECHNICAL PROBLEM

It is an object of the present invention to provide a manufacturing method of hydroxyalkanoic acid and lactic acid by hydrolyzing a hydroxyalkanoate-lactide copolymer.

### TECHNICAL SOLUTION

According to one embodiment of the present invention, there is provided a manufacturing method of hydroxyalkanoic acid and lactic acid, the method comprising the steps of: hydrolyzing a hydroxyalkanoate-lactide copolymer in the presence of an alkali metal salt to produce a hydroxyalkanoic acid inorganic salt and a lactic acid inorganic salt; and purifying the hydroxyalkanoic acid inorganic salt and the lactic acid inorganic salt by electrodialysis to manufacture hydroxyalkanoic acid and lactic acid.

Now, a manufacturing method of hydroxyalkanoic acid and lactic acid according to specific embodiments of the invention will be described in more detail.

Unless particularly mentioned herein, the term "including" or "comprising" refers to including some element (or component) without any limitation, and should not be construed as excluding addition of other elements (or components).

In addition, it should be understood that, unless steps included in a manufacturing method described herein are specified as being sequential or consecutive or otherwise stated, one step and another step included in the manufacturing method should not be construed as being limited to an order described herein. Therefore, it should be understood that an order of steps included in a manufacturing method can be changed within the range of understanding of those skilled in the art, and that, in this case, the incidental changes obvious to those skilled in the art are within the scope of the present invention.

As used herein, the term "polylactic acid prepolymer" refers to polylactic acid having a weight average molecular weight of 1,000 to 50,000 g/mol, which is obtained by oligomerizing lactic acid.

As used herein, the term "poly(3-hydroxypropionate) prepolymer" refers to poly(3-hydroxypropionate) having a weight average molecular weight of 1,000 to 50,000 g/mol, which is obtained by oligomerizing 3-hydroxypropionate.

Further, unless otherwise stated herein, the weight average molecular weight of polylactic acid prepolymer, poly(3-hydroxypropionate) prepolymer, and copolymer can be measured using a gel permeation chromatography(GPC). Specifically, the prepolymer or copolymer is dissolved in chloroform to a concentration of 2 mg/ml, then 20 *µ*ℓ of the solution is injected into GPC, and GPC analysis is performed at 40°C. At this time, chloroform is used as the mobile phase for GPC, the flow rate is 1.0 mL/min, and the column used is two Agilent Mixed-B units connected in series. RI Detector is used as a detector. The values of Mw can be obtained using a calibration curve formed using a polystyrene standard sample. Nine kinds of the polystyrene standard samples are used with the molecular weight of 2,000 g/mol, 10,000 g/mol, 30,000 g/mol, 70,000 g/mol, 200,000 g/mol, 700,000 g/mol, 2,000,000 g/mol, 4,000,000 g/mol, and 10,000,000 g/mol.

According to one embodiment of the invention, there is provided a manufacturing method of hydroxyalkanoic acid and lactic acid, the method comprising the steps of: hydrolyzing a hydroxyalkanoate-lactide copolymer in the presence of an alkali metal salt to produce a hydroxyalkanoic acid inorganic salt and a lactic acid inorganic salt; and purifying the hydroxyalkanoic acid inorganic salt and the lactic acid inorganic salt by electrodialysis to manufacture hydroxyalkanoic acid and lactic acid.

The present inventors have found that when the hydroxyalkanoate-lactide copolymer is hydrolyzed in the presence of alkali metal salts, and the lactic acid inorganic salts prepared thereby is purified by electrodialysis, a mixture of hydroxyalkanoic acid and lactic acid, which are recyclable monomers, can be recovered in high purity and high yield, and completed the present invention.

In addition, conventionally, when depolymerizing a lactide-containing copolymer, a method of hydrolyzing it using high-temperature steam was used, but this method had the disadvantage that as high-temperature steam is used, energy consumption is high and the process took a long time. However, when the hydroxyalkanoate-lactide copolymer is hydrolyzed in the presence of an alkali metal salt as in the manufacturing method of hydroxyalkanoic acid and lactic acid according to one embodiment, the process time can be shortened and the energy use can also be reduced.

However, when the hydroxyalkanoate-lactide copolymer is hydrolyzed in the presence of an alkali metal salt, a step of removing the inorganic salt is required after the hydrolysis. Ion exchange resins can be used to remove such inorganic salts, but when using an ion exchange resin, there is a problem that it is not environmentally friendly and uneconomical since the use of a large amount of water and hydrochloric acid is essentially required. However, when the hydroxyalkanoic acid inorganic salt and lactic acid inorganic salt prepared by hydrolysis are removed by electrodialysis as in the manufacturing method of hydroxyalkanoic acid and lactic acid according to the above embodiment, the amount of water and hydrochloric acid used can be significantly reduced as compared to the case of using an ion exchange resin, which may be environmentally friendly and economical.

In the manufacturing method of hydroxyalkanoic acid and lactic acid according to one embodiment, the hydroxyalkanoate-lactide copolymer may be a block copolymer that includes one or more polyhydroxyalkanoate (PHA) blocks and one or more polylactic acid blocks. As the hydroxyalkanoate-lactide copolymer contains the above-mentioned blocks, it can exhibit the environmental friendliness and biodegradable properties that polyhydroxyalkanoate and polylactic acid have.

The hydroxyalkanoate may be 3-hydroxybutyrate, 4-hydroxybutyrate, 2-hydroxypropionate, 3-hydroxypropionate, (D)-3-hydroxycarboxylate with a medium chain length of 6 to 14 carbon atoms, 3-hydroxyvalate, 4-hydroxyvalate or 5-hydroxyvalate. Therefore, the hydroxyalkanoate-lactide copolymer may be 3-hydroxybutyrate-lactide copolymer, 4-hydroxybutyrate-lactide copolymer, 2-hydroxypropionate-lactide copolymer, 3-hydroxypropionate-lactide copolymer, 4-hydroxyvalate-lactide copolymer, or 5-hydroxyvalate-lactide copolymer. For example, the hydroxyalkanoate-lactide copolymer may be 3-hydroxypropionate-lactide copolymer to improve flexibility, mechanical properties, and the like.

The 3-hydroxypropionate-lactide copolymer may be a block copolymer obtained by polymerizing a polylactic acid prepolymer and a poly(3-hydroxypropionate) prepolymer. The 3-hydroxypropionate-lactide copolymer exhibits excellent characteristic in a tensile strength and an elastic modulus that the polylactic acid prepolymer has, and also the poly(3-hydroxypropionate) prepolymer lowers the glass transition temperature(Tg), increases flexibility, and improves mechanical properties such as impact strength, thereby being able to prevent the characteristic (brittleness) of polylactic acid is that it has poor elongation and breaks easily.

The polylactic acid prepolymer may be produced by performing fermentation or polycondensation of lactic acid. The polylactic acid prepolymer may have a weight average molecular weight of 1,000 g/mol or more, or 5,000 g/mol or more, or 6,000 g/mol or more, or 8,000 g/mol or more, and 50,000 g/mol or less, or 30,000 g/mol or less. When it is desired to increase the crystallinity of the blocks containing repeating units derived from a polylactic acid prepolymer in the final produced block copolymer, it is preferred that the polylactic acid prepolymer has a high weight average molecular weight of more than 20,000 g/mol, or 22,000g/mol or more, or 23,000g/mol or more, or 25,000 g/mol or more, and 50,000 g/mol or less, or 30,000 g/mol or less, or 28,000 g/mol or less, or 26,000g/mol or less.

If the weight average molecular weight of the polylactic acid prepolymer is 20,000 g/mol or less, the crystals of the polymer are small, which makes it difficult to maintain the crystallinity of the polymer in the final produced block copolymer. If the weight average molecular weight of the polylactic acid prepolymer exceeds 50,000 g/mol, the rate of side reactions occurring within the prepolymer chain becomes faster than the reaction rate between the polylactic acid prepolymers during polymerization. On the other hand, 'lactic acid' as used herein refers to L-lactic acid, D-lactic acid, or a mixture thereof.

The poly(3-hydroxypropionate) prepolymer may be produced by performing fermentation or condensation polymerization of 3-hydroxypropionate. The weight average molecular weight of the poly(3-hydroxypropionate) prepolymer may be 1,000 g/mol or more, or 5,000 g/mol or more, or 8,000 g/mol or more, or 8,500 g/mol or more, and 50,000 g/mol or less, or 30,000 g/mol or less. When it is desired to increase the crystallinity of repeating units derived from poly(3-hydroxypropionate) prepolymer in the final produced block copolymer, it is preferable that the poly(3-hydroxypropionate) prepolymer has a high weight average molecular weight of more than 20,000 g/mol, or 22,000 g/mol or more, or 25,000 g/mol or more, and 50,000 g/mol or less, or 30,000 g/mol or less, or 28,000 g/mol or less.

As explained above for the polylactic acid prepolymer, if the weight average molecular weight of the poly(3-hydroxypropionate) prepolymer is 20,000 g/mol or less, the crystals of the polymer are small, which makes it difficult to maintain the crystallinity of the polymer in the final produced block copolymer. If the weight average molecular weight of the poly(3-hydroxypropionate) prepolymer exceeds 50,000 g/mol, the rate of side reactions occurring within the prepolymer chain becomes faster than the reaction rate between the polylactic acid prepolymers during polymerization.

That is, at least one of the polylactic acid prepolymer and the poly(3-hydroxypropionate) prepolymer may have a weight average molecular weight of more than 20,000 g/mol and 50,000 g/mol or less.

The 3-hydroxypropionate-lactide copolymer is a block copolymer obtained by polymerizing a polylactic acid prepolymer and a poly(3-hydroxypropionate) prepolymer, and the weight ratio of the polylactic acid prepolymer and the poly(3-hydroxypropionate) prepolymer in the block copolymer may be 95:5 to 50:50, 90:10 to 55:45, 90:10 to 60:40, 90:10 to 70:30, or 90:10 to 80:20. If the poly(3-hydroxypropionate) prepolymer is contained in an excessively small amount relative to the polylactic acid prepolymer, the brittleness characteristics may increase, and if the poly(3-hydroxypropionate) prepolymer is contained in an excessively large amount relative to the polylactic acid prepolymer, the molecular weight may become low, and the processability and heat resistance stability may deteriorate.

The 3-hydroxypropionate-lactide copolymer has a weight average molecular weight(Mw) of 50,000 to 300,000 g/mol, as measured using gel permeation chromatography(GPC). More specifically, the 3-hydroxypropionate-lactide copolymer has a weight average molecular weight of 50,000 g/mol or more, 70,000 g/mol or more, or 100,000 g/mol or more, and 300,000 g/mol or less, or 200,000 g/mol or less, or 150,000 g/mol or less. If the weight average molecular weight of the 3-hydroxypropionate-lactide is too small, the overall mechanical properties may be significantly reduced, and if the weight average molecular weight is too large, the process procedure may be difficult, and the processability and elongation may be lowered.

On the other hand, the lactic acid and 3-hydroxypropionate may be plastic and biodegradable compounds produced from renewable sources by microbial fermentation, and the block copolymer containing polylactic acid prepolymer and poly(3-hydroxypropionate) prepolymer formed by polymerizing them may also contain a large amount of bio raw materials while exhibiting eco-friendly property and biodegradability.

The manufacturing method of hydroxyalkanoic acid and lactic acid according to one embodiment may comprise hydrolyzing a hydroxyalkanoate-lactide copolymer in the presence of an alkali metal salt to produce a hydroxyalkanoic acid inorganic salt and a lactic acid inorganic salt.

For example, the hydroxyalkanoate-lactide copolymer, an alkali metal salt, and water may be added to a reactor, and reacted to hydrolyze the hydroxyalkanoate-lactide copolymer. The hydrolysis is performed under the alkali metal salt catalyst, thereby capable of increasing the conversion rate to hydroxyalkanoic acid and lactic acid compared to the hydrolysis performed under other catalysts, such as alkaline earth metal salts or other metal salt catalysts.

Further, the temperature during hydrolysis may be 40°C or more and 80°C or less, 45°C or more and 70°C or less, or 50°C or more and 60°C or less. If the temperature conditions during hydrolysis of the hydroxyalkanoate-lactide copolymer are too low, hydrolysis may hardly occur, and if the temperature conditions during hydrolysis are too high, the conversion rate to hydroxyalkanoic acid and lactic acid may be lowered.

Further, the pH conditions for the hydrolysis may be 11 or more and 14 or less, or 12 or more and 13 or less. If the pH conditions during hydrolysis of the hydroxyalkanoate-lactide copolymer is too low, hydrolysis may hardly occur, and if the pH conditions during hydrolysis are too high, the conversion rate to hydroxyalkanoic acid and lactic acid may decrease.

The alkali metal salt may be one selected from the group consisting of hydroxides, oxides, alkoxides, carbonates, and carboxylates of alkali metals. For example, the alkali metal salt may be one selected from the group consisting of lithium hydroxide (LiOH), sodium hydroxide (NaOH), potassium hydroxide (KOH), rubidium hydroxide (RbOH), lithium oxide (Li₂O), sodium oxide (Na₂O), potassium oxide (K₂O) and rubidium oxide (Rb₂O). Sodium hydroxide (NaOH) can be used as a catalyst for hydrolysis to improve the conversion rate and recovery rate to hydroxyalkanoic acid and lactic acid.

The alkali metal salt may be used in an amount of 0.001 parts by weight or more and 10 parts by weight or less, 0.01 parts by weight or more and 9 parts by weight or less, 0.01 parts by weight or more and 8 parts by weight or less, 0.05 parts by weight or more and 7 parts by weight or less, or 0.1 parts by weight or more and 5 parts by weight or less. If the amount of the alkali metal salt added is too small, the hydrolysis rate may become slow or the hydrolysis reaction may not proceed, and if the amount of the alkali metal salt added is too large, large amounts of impurities may be produced, or the amount of alkali metal salt used may increase, which may cause a decrease in economic efficiency.

In the manufacturing method of hydroxyalkanoic acid and lactic acid according to one embodiment, the hydroxyalkanoate-lactide copolymer is hydrolyzed in the presence of an alkali metal salt to prepare a mixture of hydroxyalkanoic acid inorganic salt and lactic acid inorganic salt, the hydroxyalkanoic acid inorganic salt may be, for example, lithium hydroxyalkanoate, sodium hydroxyalkanoate, potassium hydroxyalkanoate or rubidium hydroxyalkanoate, and the lactic acid mineral salt may be, for example, lithium lactate, sodium lactate, potassium lactate or rubidium lactate.

High-purity hydroxyalkanoic acid and lactic acid can be recovered through a purification process of removing inorganic salts from the hydroxyalkanoic acid inorganic salt and lactic acid inorganic salt prepared by the hydrolysis. In the manufacturing method of hydroxyalkanoic acid and lactic acid according to one embodiment, hydroxyalkanoic acid and lactic acid can be manufactured by purifying the inorganic hydroxyalkanoic acid and inorganic lactic acid by electrodialysis.

In the electrodialysis, the hydroxyalkanoic acid inorganic salt and the lactic acid inorganic salt can be purified by applying electricity to an electrodialysis device using a constant voltage method or a constant current method. For example, inorganic salts are removed from a solution containing inorganic hydroxyalkanoic acid and inorganic lactic acid, and at the same time, the hydroxyalkanoic acid inorganic salt and the lactic acid inorganic salt are purified and converted into hydroxyalkanoic acid and lactic acid. Thereby, a mixture of higher purity hydroxyalkanoic acid and lactic acid can be separated and recovered with high efficiency.

The electrodialysis can be performed with an electrodialysis device. Further, the electrodialysis device may include a positive electrode and a negative electrode, wherein the positive electrode and the negative electrode may be arranged at opposing positions. Further, the electrodialysis device may include a cation separator and an anion separator located between the positive electrode and the negative electrode. One cation separator and one anion separator can be included, or two or more cation separators and two or more anion separators can be included.

Further, in the electrodialysis device, the cation separator and the anion separator be arranged alternately and a desalination tank and a concentration tank can be formed by alternately arranging such cation/anion separators. Ions can be transferred by applying direct current power to the electrodialysis device in which the cation separator and the anion separator are alternately arranged, which makes it possible to alternatively form a desalination tank in which the ion concentration decreases and a concentration tank in which the ion concentration increases.

The hydroxyalkanoic acid inorganic salt and lactic acid inorganic salt may be added to the desalination tank of the electrodialysis device, and for example, a solution containing the hydroxyalkanoic acid inorganic salt and the lactic acid inorganic salt may be supplied to the desalination tank. When electric power is applied to the electrodes at both ends, the inorganic salts contained in the desalination tank may transfer to the negative electrode side due to the difference in electrical conductivity. Specifically, in the cation separator, anions can pass through, but cations cannot pass through, so that they can transfer to the concentration tank, and in the anion separator, cations pass through, but anions cannot pass through, so they can transfer to the concentration tank. Thus, in the concentration tank, the transferred inorganic salt can be concentrated and recovered. Meanwhile, the hydroxyalkanoic acid and lactic acid remain in the desalination tank, so that hydroxyalkanoic acid and lactic acid with high purity and high concentration can be finally recovered.

The step of purifying the hydroxyalkanoic acid inorganic salt and the lactic acid inorganic salt by electrodialysis to manufacture hydroxyalkanoic acid and lactic acid can be performed until the point of time at which the electrical conductivity of the desalination tank is lowered to 10% or less, 5% or less, 4% or less, 3% or less, and 1% or less relative to the initial electrical conductivity. By measuring the electrical conductivity of the desalination tank, it is possible to confirm whether or not inorganic salts have been removed.

The desalination tank may have an initial electrical conductivity of 10 mS/cm or more and 50 mS/cm or less, 15 mS/cm or more and 48 mS/cm or less, 25 mS/cm or more and 46 mS/cm or less, 28 mS/cm or more and 45 mS/cm or less, 30 mS/cm or more and 44 mS/cm or less, or 32 mS/cm or more and 42 mS/cm or less. If the initial electrical conductivity of the desalination tank is too low, it may be difficult to separate the inorganic salt to be separated, and if the initial electrical conductivity is too high, desalination may require a long time and the degree of contamination of the separator may increase.

Further, the conductivity of the desalination tank after completion of the electrodialysis may be 0.01 mS/cm or more and 3.00 mS/cm or less, 0.10 mS/cm or more and 2.00 mS/cm or less, 0.20 mS/cm or more and 1.00 mS/cm or less, 0.30 mS/cm or more and 0.50 mS/cm or less.

Further, the initial electrical conductivity of the concentrate tank may be 50 µS/cm or less, 0.1 µS/cm or more and 30 µS/cm or less, 1.0 µS/cm or more and 20 µS/cm or less, 3.0 µS/cm or more and 10 µS/cm or less. Meanwhile, the initial electrical conductivity may be the electrical conductivity at the point of time when electrodialysis begins.

The respective recovery rates of the hydroxyalkanoic acid and lactic acid may be 30% or more, 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%. The recovery rate may be calculated on a weight basis.

The respective purities of the hydroxyalkanoic acid and lactic acid may be 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, to 90 to 95%.

### ADVANTAGEOUS EFFECTS

According to the present invention, it is possible to provide a manufacturing method of hydroxyalkanoic acid and lactic acid, in which the hydroxyalkanoate-lactide copolymer is hydrolyzed and converted into hydroxyalkanoic acid and lactic acid with high purity and high yield by an environmentally friendly and economical method.

### DETAILED DESCRIPTION OF THE EMBODIMENTS

Hereinafter, the invention will be described in more detail with reference to the following examples. However, these examples are presented for illustrative purposes only, and the scope of the invention is not limited thereby in any way.

### Preparation Example 1: Preparation of 3-hydroxypropionate-lactide block copolymer

### (1) Preparation of polylactic acid prepolymer

25 g of 85% L-lactic acid aqueous solution was added to a 100 ml Schlenk flask in the oil bath, and the pressure was reduced at 70°C and 50 mbar for 2 hours to remove moisture in L-lactic acid. Subsequently, based on 100 parts by weight of lactate, 0.4 parts by weight of p-toluenesulfonic acid (p-TSA) and 0.1 part by weight of SnCl₂ catalyst were added, respectively, and a melt condensation polymerization reaction was carried out at a temperature of 150°C for 12 hours. After the reaction was completed, the reaction product was dissolved in chloroform and then extracted with methanol to obtain a polylactic acid prepolymer (weight average molecular weight: 8,000 g/mol).

### (2) Preparation of poly(3-hydroxypropionate) prepolymer

25 ml of 60% 3-hydroxypropionate aqueous solution was added to a 100 ml Schlenk flask in an oil bath, moisture in 3-hydroxypropionate was removed at 50°C and 50 mbar for 3 hours. Subsequently, oligomerization was carried out at 70°C and 20 mbar for 2 hours, 0.4 parts by weight of p-toluenesulfonic acid (p-TSA) catalyst based on 100 parts by weight of 3-hydroxypropionate was added to a reaction flask, and a melt condensation polymerization reaction was carried out at a temperature of 110°C for 24 hours. After the reaction was completed, the reaction product was dissolved in chloroform and then extracted with methanol to obtain poly(3-hydroxypropionate) prepolymer (weight average molecular weight: 26,000 g/mol).

### (3) Preparation of block copolymer

The polylactic acid prepolymer and the poly(3-hydroxypropionate) prepolymer were mixed at a weight ratio of 8:2 in a 100 ml Schlenk flask in an oil bath, and the mixture was added thereto so that the total content was 30 g, and then 90 mg of p-toluenesulfonic acid (p-TSA) was added, and annealed at 60°C for 3 hours. Subsequently, they were mixed using an evaporator at 150°C and 0.5 mbar for 24 hours and subjected to solid phase polymerization reaction to produce a 3-hydroxypropionate-lactide block copolymer (weight average molecular weight: 130,000 g/mol).

On the other hand, the weight average molecular weight of the polylactic acid prepolymer, poly(3-hydroxypropionate) prepolymer, and block copolymer was measured using a gel permeation chromatography (GPC).

### Preparation Example 2: Preparation of 3-hydroxypropionate-lactide block copolymer

3-Hydroxypropionate-lactide block copolymer was prepared in the same manner as in Preparation Example 1, except that the polylactic acid prepolymer and the poly(3-hydroxypropionate) prepolymer were mixed at a weight ratio of 5:5.

### <Example and Comparative Example>

### Example 1

10 g of 3-hydroxypropionate-lactide block copolymer prepared in Preparation Example 1 and 200 ml of water were added to a reactor and stirred, and an aqueous solution of sodium hydroxide (NaOH, concentration 40%) was gradually added so as to have pH 12. Then, the temperature inside the reactor was heated to 50°C, the mixture was stirred for 1 hour, and the temperature of the product was cooled to 25°C.

Then, the hydrolyzed product was transferred to a desalination tank of the electrodialysis device and subjected to electrodialysis to remove inorganic salts (Na⁺) from the product, and a mixture of 3-hydroxypropionic acid, lactic acid and acrylic acid was recovered. At this time, the electrodialysis conditions are as follows.

### <Electrodialysis conditions>

Stack: Innomeditech L3 membrane Type 10, 20 cells, constant voltage method (20 V, 1V per cell)
Concentration tank: Initial electrical conductivity is 5 µS/cm
Desalination tank: initial electrical conductivity is 42 mS/cm
Desalination tank: Electrodialysis was performed unti1 the point of time at which the electrical conductivity was lowered to 1% relative to the initial conductivity.
Flux: 10

### Example 2

Inorganic salts (Na⁺) were removed from the product to recover a mixture of 3-hydroxypropionic acid, lactic acid, and acrylic acid in the same manner as in Example 1, except that 10 g of the 3-hydroxypropionate-lactide block copolymer prepared in Preparation Example 2 was used.

### Comparative Example 1

1 g of poly(3-hydroxypropionate) and 20 ml of water were added to a reactor and stirred, and an aqueous solution of sodium hydroxide (NaOH, concentration 40%) was gradually added so as to have pH 12. Then, the temperature inside the reactor was heated to 50°C, the mixture was stirred for 1 hour, and the temperature of the product was cooled to 25°C.

Then, inorganic salts (Na⁺) were removed from the hydrolyzed product using a cation exchange resin (TRILITE^{®} CMP-28LH, Samyang Corporation) and concentrated to recover 3-hydroxypropionic acid with a concentration of 30% was recovered.

### Comparative Example 2

50 g of polylactic acid (PLA, NatureWorks) and 500 ml of water were added to a reactor and stirred, and an aqueous solution of sodium hydroxide (NaOH, concentration 20%) was gradually added so to have pH 12. Then, the temperature inside the reactor was heated to 80°C, and the mixture was stirred. After hydrolysis was completed, the temperature of the product was cooled to 25°C. Then, the conversion rate to lactic acid was confirmed by ¹H NMR (conversion rate 99%).

Then, the hydrolyzed product was transferred a desalination tank of an electrodialysis device and subjected to electrodialysis to remove inorganic salts (Na⁺) from the product. At this time, the electrodialysis conditions are as follows. In addition, the solution recovered from the desalination tank from which the inorganic salts have been removed was concentrated, and 51 g of an aqueous lactic acid solution with a concentration of 92% was recovered (47 g of lactic acid in the aqueous solution, recovery rate 94%).

### <Electrodialysis condition>

Stack: Innomeditech L3 membrane Type 10, 20 cells, constant voltage method (20 V, 1V per cell)
Concentration tank: Initial electrical conductivity is 10 µS/cm
Desalination tank: Electrodialysis was performed until the point of time at which the electrical conductivity was lowered to 1% relative to the initial conductivity.
Flux: 27.3

### Evaluation

### 1. Evaluation of conversion rate, product composition and inorganic salt content

The conversion rate, product composition and inorganic salt content of the products recovered from Examples and Comparative Examples were analyzed by high-performance liquid chromatography (HPLC), and are shown in Table 1 below.

The conversion rate refers to the conversion ratio of each polymer of 3-hydroxypropionate-lactide block copolymer and poly(3-hydroxypropionate) to a monomer such as 3-hydroxypropionic acid, and the product composition refers to the proportion (wt.%) of monomers such as 3-hydroxypropionic acid, lactic acid, and acrylic acid in 100 wt.% of the final product.

### <HPLC analysis conditions>

Column: Aminex HPX-87H ion exclusion column (7.8 X 300 mm, 9 um)
Mobile phase: 0.5 mM sulfuric acid
Flow rate: 0.4 ml/min
Column temperature: 35°C
Injection volume: 5 uL
Detector: UV 210 nm

**[Table 1]**

| | Conversion rate (%) | Product composition (wt.%) | | | Inorganic salt content (ppm) | | | | |
|---|---|---|---|---|---|---|---|---|---|
| | | 3-hydroxypropionic acid | Lactic acid | Acrylic acid | Ca | Na | K | S | Cl |
| Example 1 | 99 | 16 | 72 | 4 | <10 | 115 | <10 | <10 | <10 |
| Example 2 | 99 | 41 | 47 | 9 | <10 | 210 | <10 | <10 | <10 |
| Comparative Example 1 | 99 | 80 | - | 20 | <10 | <10 | <10 | 17 | <30 |
| Comparative Example 2 | 99 | - | 100 | - | <10 | 190 | <10 | <10 | 38 |

According to Table 1, it was confirmed that in Example 1, large amounts of 3-hydroxypropionic acid and lactic acid were produced, and also the content of impurities was small. On the other hand, it was confirmed that in Comparative Example 1, only 3-hydroxypropionic acid was produced, and particularly, a large amount of the 3-hydroxypropionic acid was converted to acrylic acid, and in Comparative Example 2, only lactic acid was produced.

## Claims

1. A manufacturing method of a hydroxyalkanoic acid and a lactic acid, the method comprising the steps of:
hydrolyzing a hydroxyalkanoate-lactide copolymer in the presence of an alkali metal salt to produce a hydroxyalkanoic acid inorganic salt and a lactic acid inorganic salt; and
purifying the hydroxyalkanoic acid inorganic salt and the lactic acid inorganic salt by electrodialysis to manufacture the hydroxyalkanoic acid and the lactic acid.

2. The manufacturing method according to claim 1, wherein:
the hydroxyalkanoate-lactide copolymer is a 3-hydroxypropionate-lactide copolymer.

3. The manufacturing method according to claim 2, wherein:
the 3-hydroxypropionate-lactide copolymer is a block copolymer obtained by polymerizing a polylactic acid prepolymer and a poly(3-hydroxypropionate) prepolymer.

4. The manufacturing method according to claim 1, wherein:
the hydroxyalkanoic acid is 3-hydroxypropionic acid.

5. The manufacturing method according to claim 1, wherein:
the hydrolysis step is performed at a temperature of 40°C or more and 80°C or less.

6. The manufacturing method according to claim 1, wherein:
the hydrolysis step is performed at pH of 11 or more and 14 or less.

7. The manufacturing method according to claim 1, wherein:
the alkali metal salt is used in an amount of 0.001 parts by weight or more and 10 parts by weight or less based on 100 parts by weight of the hydroxyalkanoate-lactide copolymer.

8. The manufacturing method according to claim 1, wherein:
the alkali metal salt is one selected from the group consisting of hydroxides, oxides, alkoxides, carbonates, and carboxylates of alkali metals.

9. The manufacturing method according to claim 1, wherein:
the alkali metal salt is sodium hydroxide.

10. The manufacturing method according to claim 1, wherein:
the electrodialysis is performed in an electrodialysis device, and
the electrodialysis device comprises,
a positive electrode and a negative electrode;
a cation separator and an anion separator located between the positive electrode and the negative electrode; and
a desalination tank and a concentration tank formed by alternately arranging the cation separator and the anion separator.

11. The manufacturing method according to claim 10, wherein:
the hydroxyalkanoic acid inorganic salt and the lactic acid inorganic salt are added to the desalination tank of the electrodialysis device.

12. The manufacturing method according to claim 10, wherein:
an initial electrical conductivity of the concentration tank is 50 µS/cm or less.

13. The manufacturing method according to claim 10, wherein:
the step of purifying the hydroxyalkanoic acid inorganic salt and the lactic acid inorganic salt by electrodialysis to manufacture hydroxyalkanoic acid and lactic acid,
is performed until the point of time at which an electrical conductivity of the desalination tank is lowered to 10% or less relative to an initial electrical conductivity.

14. The manufacturing method according to claim 13, wherein:
the initial electrical conductivity of the desalination tank is 10 mS/cm or more and 50 mS/cm or less.
